Europäisches Patentamt

European Patent Office

Office européen des brevets

(11) Veröffentlichungsnummer: **0 282 021**
**A2**

# (12) EUROPÄISCHE PATENTANMELDUNG

(21) Anmeldenummer: 88103697.4

(22) Anmeldetag: 09.03.88

(51) Int. Cl.⁴: **C07K 15/00** , C07K 3/18 ,
C07K 17/14 , C12P 21/00 ,
G01N 33/68 , G01N 33/577 ,
//(C12P21/00,C12R1:91)

(30) Priorität: 11.03.87 DE 3707746

(43) Veröffentlichungstag der Anmeldung:
14.09.88 Patentblatt 88/37

(84) Benannte Vertragsstaaten:
AT BE CH DE ES FR GB GR IT LI LU NL SE

(71) Anmelder: BOEHRINGER MANNHEIM GMBH
Sandhofer Strasse 116
D-6800 Mannheim 31(DE)

(72) Erfinder: Hallermayer, Klaus, Dr. rer. nat.
Von-der-Tann-Strasse 9
D-8130 Starnberg(DE)
Erfinder: Looser, Siegfried, Dr. rer. nat.
Ammerstrasse 27
D-8120 Weilheim(DE)
Erfinder: Katus, Hugo, Dr. med.
Lachenweg 6
D-6901 Bammental(DE)

(54) Spezifische Antikörper gegen Herzmuskelmyosin-Leichtketten, ihre Herstellung und Verwendung in einem Reagenz zur Bestimmung von Herzmuskelmyosin-Leichtketten.

(57) Spezifische Antikörper gegen Herzmuskelmyosin-Leichtketten (HMLC) mit einer Kreuzreaktivität von unter 5 % gegenüber SMLC, ihre Herstellung sowie ein Reagenz zur Bestimmung von HMLC. Durch Reinigung eines polyklonalen Antiserums über Immunsorption an einem SMLC-Immunadsorbens können Antikörper mit derartig geringer Kreuzreaktivität hergestellt werden.

Ein Reagenz und ein Verfahren zur Bestimmung von HMLC, ggf. unter Verwendung von monoklonalen Antikörpern zeigen eine besonders gute Empfindlichkeit und einen geringen Probenleerwert.

EP 0 282 021 A2

## Spezifische Antikörper gegen Herzmuskelmyosin-Leichtketten, ihre Herstellung und Verwendung in einem Reagenz zur Bestimmung von Herzmuskelmyosin-Leichtketten

Die Erfindung betrifft spezifische Antikörper gegen Herzmuskelmyosin-Leichtketten (HMLC), deren Herstellung und Verwendung in einem immunologischen Reagenz zur Bestimmung von HMLC.

Myosin ist ein Protein mit einem Molekulargewicht von ca. 500.000. Es besteht aus zwei schweren Ketten mit einem Molekulargewicht von ca. 200.000, zwei leichten Ketten I (HMLC I) mit einem Molekulargewicht von ca. 26.000 und zwei leichten Ketten II (HMLC II) mit einem Molekulargewicht von ca. 18.000. Myosin liegt in den dicken Filamenten der Herz-und Skelettmuskulatur vor.

Durch infarktbedingte Herzmuskelnekrosen gelangen aus dem Abbau von Myosin HMLC in das Blutplasma und stellen somit einen spezifischen Parameter dar, der nicht nur zur Diagnostik und Verlaufskontrolle eines Myokardinfarkts geeignet erscheint, sondern der auch über das Ausmaß der Herzmuskelschädigung Aufschluß geben kann (Circulation 58 [1968] 1130, Jap. Cardiol. J. 44 [1980] 185, Am. J. Cardiol. 41 [1978] 641, Circulation 60 [Suppl. II] [1979] 139).

Da die Halbwertszeit von HMLC im Plasma mit ca. 70 - 80 min sehr gering ist, läßt ein später Abfall nach dem Infarktereignis bzw. eine breite Verlaufskurve auf anhaltende Freisetzung von HMLC vermuten. Damit ist im Gegensatz zu den sonst bestimmten "Infarktenzymen" CK, CK-MB, GOT und LDH mit der Bestimmung von HMLC eine zuverlässigere Diagnostik und Verlaufskontrolle möglich. Außerdem scheinen auch zuverlässigere Aussagen zur Beurteilung der instabilen Angina pectoris möglich, da in derartigen Fällen durch möglicherweise vorhandene Myokardinfarkte ebenfalls HMLC ausgeschwemmt werden (Am. J. Cardiol. 54 [1984] 964). Da allerdings hierbei nur geringe HMLC-Mengen in das Blutplasma gelangen, ist eine sensitive und spezifische Bestimmungsmethode erforderlich. Üblicherweise eignen sich in derartigen Fällen insbesondere immunologische Bestimmungsmethoden als Nachweismethoden.

Ein Radioimmunoassay für HMLC ist bereits in Am. J. Clin. Path. 71 (1978) 309 - 318 beschreiben. Hierbei handelt es sich um einen kompetitiven Radioimmunoassay, bei dem HMLC aus der Probe und jodiertes HMLC um polyklonale Antikörper, die an eine Festphase adsorbiert sind, konkurrieren. Der Nachteil dieses Verfahrens liegt jedoch darin, daß die Kreuzreaktivität zwischen HMLC und Skelettmuskel-Leichtketten (SMLC) für das verwendete Antiserum bei 6,8 % liegt. Es wird zwar vorgeschlagen, das Antiserum gegen HMLC durch Entfernen der SMLC-Antikörper oder durch Fraktionierung mit Affinitätschromatographie zu reinigen, jedoch konnten auch dadurch bisher keine Antikörper gegen HMLC zur Verfügung gestellt werden, die eine ausreichend geringe Kreuzreaktivität mit SMLC zeigen. So wurde in Jap. Circulation 44 (1980) 185 - 186 im immunologischen Sandwichtest eine Kreuzreaktivität von 8 % festgestellt. In Circulation 58 (1978) 1130 - 1136 wird im immunologischen Sandwichtest eine Kreuzreaktivität von 2,9 - 10 % berichtet. Die tatsächliche Kreuzreaktivität der Antikörper ist demnach wesentlich höher. Für den im immunologischen Sandwichtest gemessenen Wert von 2,9 % beträgt sie $(2,9)^2$ %, d. h. 8,4 %.

In Molecular Immunology 19 (1982) 451 - 455 wird versucht, diesen Nachteil dadurch zu überwinden, daß anstelle von polyklonalen Antikörpern monoklonale Antikörper eingesetzt werden. Jedoch zeigen auch diese Antikörper nur eine unbefriedigende Kreuzreaktivität (17 %). Auch die in EP 205177 beschriebenen monoklonalen Antikörper sind für die spezifische HMLC-Bestimmung nicht geeignet. Alle dort beschriebenen Antikörper, die mit HMLC aus Patientenserum reagieren, reagieren auch in erheblichem Umfang mit SMLC.

Die Aufgabe der vorliegenden Erfindung besteht darin, die beschriebenen Nachteile zu überwinden und Antikörper gegen HMLC mit einer geringen Kreuzreaktivität gegenüber SMLC sowie ein Verfahren zur Herstellung derartiger Antikörper zur Verfügung zu stellen.

Gegenstand der Erfindung sind Antikörper gegen HMLC mit einer Kreuzreaktivität gegenüber SMLC von unter 5 %, vorzugsweise unter 2 % und besonders bevorzugt unter 1 %. Derartige Antikörper sind nach dem nachstehend beschriebenen erfindungsgemäßen Verfahren erhältlich.

Ein weiterer Gegenstand der Erfindung ist ein Verfahren zur Herstellung von Antikörpern gegen HMLC durch Immunisierung einer Tierspezies mit humanem HMLC und Gewinnung des Rohserums dadurch gekennzeichnet, daß das Rohserum, ggf. nach einer Vorreinigung, über eine negative Immunsorption an einem SMLC-Immunadsorbens auf Silicatbasis gereinigt wird.

Überraschenderweise hat sich gezeigt, daß es mit dem erfindungsgemäßen Verfahren gelingt, Antikörper gegen HMLC zu erhalten, deren Kreuzreaktivität gegen SMLC unter 5 %, vorzugsweise unter 2 % und besonders bevorzugt unter 1 % liegt.

Die Bestimmung der Kreuzreaktivität erfolgte bei Verwendung von HMLC bzw. SMLC als Festphase und maximal 0,1 μg Antikörper gegen HMLC pro Probe. Die Nachweisreaktion erfolgte über ein Konjugat aus einem gegen den Fcγ-Teil des HMLC-Antikörpers gerichteten Antikörper und einer bestimmbaren Gruppe (z. B. POD).

HMLC und SMLC können nach den dem Fachmann geläufigen Methoden, wie sie beispielsweise in Circul. Res. 19 (1985) 611 und Biochem. J. 119 (1970) 31 beschrieben sind, aus Herz-bzw. Skelettmuskel isoliert werden. Zur Immunisierung kann HMLC I, HMLC II oder ein Gemisch aus HMLC I und II als Immunogen verwendet werden. Die Immunisierung kann an jeder beliebigen Tierspezies und nach den dem Fach mann geläufigen Methoden erfolgen.

Das hierbei erhaltene Rohserum kann direkt zur negativen Immunsorption an einem SMLC-Immunadsorbens auf Silicatbasis eingesetzt werden.

Vorzugsweise wird jedoch zunächst eine Vorreinigung, beispielsweise über Ionenaustauscherchromatographie und Dialyse, durchgeführt.

Das ggf. so vorbehandelte Rohserum wird einer negativen Immunsorption an einem SMLC-Immunadsorbens auf Silicatbasis unterworfen. Hierzu wird das Rohserum auf die Säule aufgegeben, wobei überraschenderweise diejenigen Antikörper gegen HMLC, die eine Kreuzreaktivität unter 5 % haben, vorzugsweise unter 2 % und besonders bevorzugt unter 1 %, nicht gebunden werden, während die Antikörper mit höherer Kreuzreaktivität gebunden werden. Anschließend wird die Säule mit den hierzu üblichen Reagenzien (z. B. Phosphatpuffer pH 7,5, PBS) proteinfrei gewaschen.

Das so erhaltene Eluat kann direkt zur Herstellung der Reagenzien für einen HMLC-Test verwendet werden. Vorzugsweise wird jedoch das Eluat einer weiteren Reinigung, insbesondere durch positive Immunsorption an einem HMLC-Immunadsorbens, unterworfen.

Als Träger für das SMLC-Immunadsorbens sind die dem Fachmann geläufigen Sorbentien auf Silicatbasis geeignet. Die Medien können beispielsweise als Gel, in pulverisierter oder granulierter Form vorliegen. Geeignete Träger sind beispielsweise Spherosil® (Hersteller: Rhône-Poulenc), SelectiSper® - (Pierce Eurochemie) oder Silicagele von J. T. BakerChem. An den Träger wird nach den üblichen Verfahren SMLC, ggf. über einen Spacer/Linker, kovalent gebunden.

Um die Bindung von SMLC an die Silicate zu ermöglichen, müssen diese zweckmäßig mit bindefähigen Gruppen versehen werden. Vorzugsweise setzt man hierzu die Silicate mit reaktiven Silanen, wie z. B. Trichloraminopropylsilan oder 3-(Triäthoxysilyl)propylamin um. An die hierdurch am Träger entstandenen funktionellen Gruppen kann dann direkt SMLC gekoppelt werden. In einer bevorzugten Ausführungsform wird an die funktionelle Gruppe zunächst ein bifunktionelles Reagenz als Spacer gekoppelt und anschließend daran SMLC gebunden. Als vorteilhaft hat sich hierbei die Verwendung eines Gemischs aus SMLC I und SMLC II erwiesen. Das Verhältnis SMLC I : SMLC II ist hierbei unkritisch. Vorzugsweise wird jedoch ein Verhältnis von annähernd 1 : 1 oder ein Überschuß von SMLC I verwendet.

Als Medien besonders geeignet sind: Silicate, welche Aminopropyl-, Succinoylaminopropyl-, Hexamethylendiamin-, Diol-, Diazafluoroborat-, p-Nitrophenyl-, Epoxypropyl-, Glutaraldehyd-und/oder Glycidoxypropylgruppen tragen. Ggf. wird vor der Kupplung an das Trägermaterial ein Spacer/Linker eingeführt. Geeignet sind beispielsweise die dem Fachmann geläufigen bifunktionellen Linker, wie z. B.:
Glutardialdehyd,
N-succinimidyl 3-(2-pyridyldithio)propionate,
Ethyl 4-azidophenyl-1,4-dithiobutyrimidate x HCl,
4-Fluoro-3-nitrophenyl azide,
N-Hydroxysuccinimidyl-4-azidobenzoate,
m-Maleimidobenzoyl-N-hydroxysuccinimide ester,
N-succinimidyl-6(4'-azido-2'-nitrophenyl-amino)hexanoate,
N-Succinimidyl(4-iodacetyl)aminobenzoate oder
Succinimidyl-4-(-p-maleimidophenyl)butyrate.

Es erweist sich als vorteilhaft, wenn der Abstand zwischen Silicatträger und SMLC nicht mehr als 20 Atome beträgt. Als besonders vorteilhaft hat sich ein Abstand von 7 - 12 Atomen erwiesen.

Als besonders vorteilhaft hat sich erwiesen, wenn man als Silicat Spherosil, welches mit Trichloraminopropylsilan oder 3-(Triäthoxysilyl)propylamin aktiviert ist, verwendet, dieses Aminospherosil mit Glutardialdehyd als bifunktionellem Reagenz umsetzt und SMLC an den so vorbereiteten Träger bindet.

Es erweist sich als vorteilhaft, nach der Reinigung über die SMLC-Säule, ggf. unter Zwischenschaltung eines Dialyseschrittes, eine weitere immunsorptive Reinigung an einem HMLC-Immunadsorbens anzuschließen. Als Träger sind die dem Fachmann geläufigen Träger geeignet. Besonders vorteilhaft ist es jedoch, wenn auch hier ein Immunadsorbens auf Silicatbasis verwendet wird. Für die Kupplung von HMLC an diesen Träger gelten die Ausführungen zu dem SMLC-Immunadsorbens sinngemäß.

Ein weiterer Gegenstand der Erfindung ist ein immunologisches Reagenz zur Bestimmung von HMLC unter Verwendung von HMLC-Antikörpern mit einer Kreuzreaktivität von unter 5 %, vorzugsweise unter 2 %, besonders bevorzugt unter 1 % gegenüber SMLC.

Mit einem derartigen Reagenz können beispielsweise Sandwichtests oder kompetitive Tests durchgeführt werden.

In einem Sandwichtest wird ein immobilisierter Antikörper gegen HMLC und ein Konjugat aus einem HMLC-Antikörper und einer bestimmbaren Gruppe verwendet. Hierbei kann entweder nur einer oder beide der zu verwendenden Antikörper nach dem erfindungsgemäßen Verfahren hergestellt sein.

Unter einer bestimmbaren Gruppe sind die dem Fachmann geläufigen Gruppe, wie z. B. Enzym, Fluoreszenzfarbstoff und radioaktive Markierung, zu verstehen.

Mit einem derartigen Sandwichtest gelingt es bei Inkubationszeiten von unter 5 h, noch HMLC-Konzentrationen von 2 ng/ml zu erfassen, ohne daß eine Kreuzreaktivität mit SMLC gemessen werden kann.

Das Reagenz kann sowohl in Lösung als auch auf bzw. in einem Trockenreagenzträger vorliegen. Da auf einem Trockenreagenzträger eine Verdünnung der Probe nicht erforderlich ist, können hiermit kürzere Inkubationszeiten erreicht werden.

Eine bevorzugte Ausführungsform des Reagenzes enthält einen nach dem erfindungsgemäßen Verfahren hergestellten und immobilisierten Antikörper gegen HMLC und ein Konjugat aus einem monoklonalen HMLC-Antikörper und einer bestimmbaren Gruppe. Überraschenderweise hat sich gezeigt, daß ein Reagenz, welches eine derartige Kombination enthält, im Test eine hohe Empfindlichkeit und einen geringen Probenleerwert bei einer HMLC-Bestimmung ergibt.

Die Herstellung der monoklonalen Antikörper erfolgt nach den dem Fachmann geläufigen Methoden nach Immunisierung mit HMLC. Als besonders geeignet erweisen sich die Antikörper MAK 4D12 und 1B10. Die Zellinien, die diesen monoklonalen Antikörpern entsprechen, sind bei der European Collection of Animal Cell Cultures (ECACC), Porton Down, GB, unter den Hinterlegungsnummern ECACC 88022503 (MAK 4D12) und ECACC 88022504 (MAK 1B10) hinterlegt.

Ein weiterer Gegenstand der Erfindung ist ein Verfahren zur Bestimmung von HMLC, welches dadurch gekennzeichnet ist, daß die Probe mit einem erfindungsgemäß hergestellten Antikörper gegen HMLC, der immobilisiert ist, und einem Konjugat aus einem monoklonalen Antikörper und einer bestimmbaren Gruppe zusammengebracht wird. Dabei ist die Reihenfolge der Zugabe beliebig. Ggf. nach einer Inkubation werden feste und flüssige Phase getrennt und die bestimmbare Gruppe in einer der beiden Phasen bestimmt.

Die nachfolgenden Beispiele und die Abbildung erläutern die Erfindung weiter.

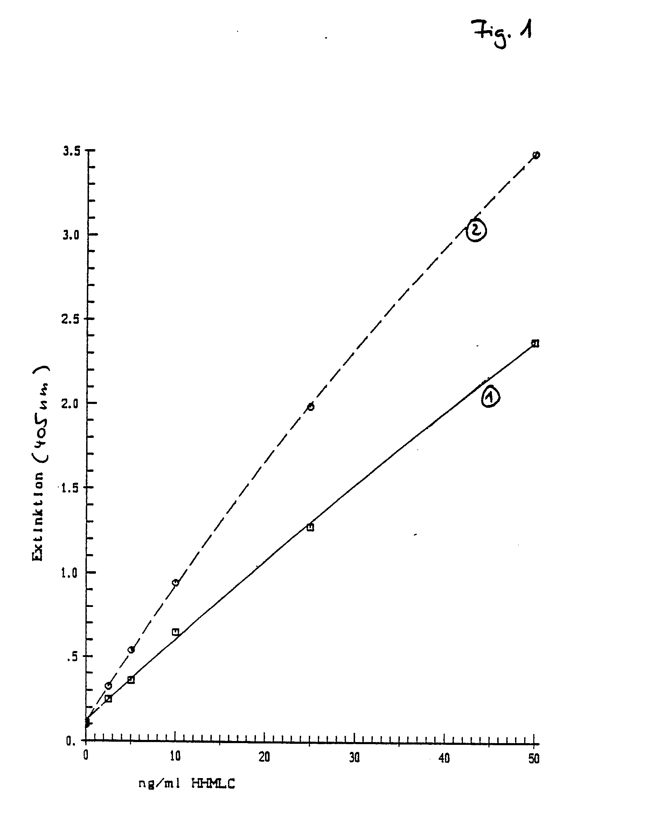

Fig. 1 zeigt eine Eichkurve für einen ELISA-Sandwichtest (nach Bsp. 9) mit zwei verschiedenen MAKs.
Kurve 1: MAK 4D12
Kurve 2: MAK 1B10

## Beispiel 1

Herstellung und Isolierung der Mysoin-Leichtketten (HMLC und SMLC)

HMLC und SMLC wurden nach der in Circul. Res. 19 (1965) 611 und Biochem. J. 119 (1970) 31 beschriebenen Verfahren aus Human-Skelett-und -Herzmuskelgewebe isoliert.

## Beispiel 2

Gewinnung der Antiseren

Eine Mischung von HMLC I (MG 26.000) und HMLC II (MG 18.000) wurde als Immunogen verwendet. Schafe wurden mit 55 µl einer Lösung von 0,1 mg/ml HMLC I und II in komplettem Freud'schen Adjuvans (CFA) erstimmunisiert. Weitere Immunisierungen erfolgten am 7., 14. und 30. Tag. Im weiteren wurde alle 30 Tage immunisiert. Bei diesen Folgeinjektionen wurden jeweils 28 ml einer HMLC-Lösung von 0,05 mg/ml in CFA verwendet. Nach 6 Monaten wurde das Rohserum gewonnen.

## Beispiel 3

Isolierung von polyklonalen Schaf-IgG-Antikörpern gegen HMLC (PAK<HMLC>S-IgG) aus Rohserum

1 l Rohserum wird mit 15 g Aerosil (Hersteller: Degussa) versetzt, 1 h bei Raumtemperatur gerührt und zentrifugiert. Anschließen wird der Überstand mit 1,7 mol/l Ammonsulfat versetzt und 2 h bei Raumtemperatur langsam gerührt. Anschließend wird der Niederschlag abzentrifugiert und in 0,2 l Dialysepuffer (15 mmol/l Kaliumphosphat, 50 mmol/l Natriumchlorid, pH 7,0) homogenisiert und bei 4 °C gegen 4mal 10 l Dialysepuffer dialyisiert.

Nach erneuter Zentrifugation wird das dialysierte γ-Globulin über DE52-Zellulose unter Elution mit Dialysepuffer gereinigt.

Die Kreuzreaktivität des so gereinigten PAK<HMLC> gegenüber SMLC beträgt bis zu 100 % (Bestimmung nach Beispiel 6).

**Beispiel 4**

Negative Immunadsorption von PAK<HMLC> an SMLC-Immunadsorbens

Die nach Beispiel 3 gereinigte PAK<HMLC>-Lösung wird ad 50 mmol/l Kaliumphosphat pH 7,5; 150 mmol/l Natriumchlorid (PBS); 0,1 % Azid aufgestockt und mit einer Konzentration von ca. 15 mg/ml bei Raumtemperatur über eine SMLC-Immunadsorbens-Säule (Herstellung Bsp. 8) gegeben und mit PBS/0,1 % Azid proteinfrei gewaschen (PBS: phosphate buffered saline).

**Beispiel 5**

Positive Immunsorption von PAK<HMLC> an HMLC-Immunadsorbens

Das nach Beispiel 4 erhaltene Eluat wird unter den in Beispiel 4 beschriebenen Bedingungen auf ein HMLC-Immunadsorbens aufgetragen (Herstellung analog Bsp. 8). Nach Waschen mit PBS/0,1 % Azid wird mit 1 mol/l Propionsäure bei Raumtemperatur eluiert.

Das Eluat wird anschließend 2 h bei 4 °C gegen VE-Wasser und danach bei 4 °C über Nacht gegen 1 mmol/l Carbonat/Bicarbonatpuffer (pH 9,5) dialysiert.

**Beispiel 6**

Bestimmung der Kreuzreaktivität

Zur Bestimmung der Kreuzreaktivität wurde ein Testsystem, bestehend aus immobilisiertem HMLC und einem polyklonalen Antikörper gegen Schaf-Fcγ (<Schaf-Fcγ>-PAK), konjugiert an POD, verwendet.

a) Herstellung der Festphase

Als Träger wurden Mikrotiterplatten der Fa. Costar (PVC) verwendet. Pro cavity wurden 0,1 ml einer Lösung von 5 μg/ml HMLC in 0,2 mol/l Carbonatpuffer pH 9,4 gegeben und bei 4 °C über Nacht inkubiert. Nach 2maligem Waschen mit PBS wurde mit 0,2 ml 50 mmol/l Phosphatpuffer pH 7,4, der 1 % RSA und 0,9 % NaCl enthielt, pro cavity zugegeben und 1 h bei Raumtemperatur inkubiert.

b) Herstellung des POD-Konjugats

Ein polyklonaler Antikörper aus Kaninchen gegen Fcγ (IgG) [<Schaf-Fcγ>-PAK] wird nach Meth. in Enzymology 70 (1980) 104 - 142 an Meerrettich-POD (Peroxidase) gekoppelt.

### c) Durchführung der Bestimmung

Die Probe wird mit 50 mmol/l Phosphatpuffer pH 7,4 verdünnt auf 1,0 µg/ml PAK<HMLC> nach Bsp. 3 und 4 bzw. 0,1 µg/ml PAK<HMLC> nach Bsp. 5. 0,1 ml der so aufbereiteten Probe werden pro cavity zugegeben und 1 h bei Raumtemperatur inkubiert. Nach 2maligem Waschen mit PBS werden 0,1 ml pro cavity <Schaf-Fcγ>-PAK-POD-Konjugat (POD-Aktivität 0,2 U/ml) zugegeben und 1 h bei Raumtemperatur inkubiert.

Nach 2maligem Waschen mit PBS werden 0,1 ml pro cavity ABTS® (2,2'-Azino-di-[3-ethyl-benzthiazolin-sulfonsäure(6)]-diammoniumsalz) (1,9 mmol/l in 100 mmol/l Phosphat-/Citratpuffer pH 4,4, 3,2 mmol/l Natriumperborat) zugegeben. Nach einer Inkubationszeit von 45 min wird die Extinktionsdifferenz gegen den Leerwert bei 405 nm bestimmt. Die Kreuzreaktivität wird ermittelt aus den Extinktionswerten, die für die gleiche Probe mit einer HMLC-und einer SMLC-beschichteten Festphase erhalten werden.

Hierbei ergab sich, daß nach negativer Immunsorption an SMLC und positiver Immunsorption an HMLC keine Kreuzreaktivität des PAK<HMLC> gegenüber SMLC nachweisbar ist (Tabelle 1).

Tabelle 1:

| Probe | Extinktionsdifferenz ΔE | | Kreuzreak-tivität $^o/_o$ |
| --- | --- | --- | --- |
| | HMLC-Festphase | SMLC-Festphase | |
| PAK nach Bsp. 3 | 0,752 | 0,500 | 66 |
| PAK nach Bsp. 4 (IS an SMLC-Säule) | 0,440 | 0,008 | 1,8 |
| PAK nach Bsp. 5 (IS an SMLC-und HMLC-Säule) | 0,852 | 0,0 | 0 |

IS: Immunsorption

## Beispiel 7

HMLC-Bestimmung über ELISA-Sandwich-Test

Für die Bestimmung wurde ein Testsystem verwendet, welches aus einem immobilisierten <HMLC>-PAK (hergestellt nach Beispiel 5) und einem Konjugat aus einem <HMLC>-PAK und POD bestand (Konjugatherstellung analog Beispiel 6 b).

a) Festphase: PVC-Mikrotiterplatten, Fa. Costar

b) Beschichtung (Immobilisierung von <HMLC>-PAK): 0,1 ml/cavity
5 µg/ml <HMLC>-PAK (Beispiel 5) in 0,1 mol/l Citratpuffer pH 4,0 über Nacht bei 4 °C inkubieren und 2mal mit PBS (phosphat buffered saline) waschen.

c) Nachbeladung: 0,2 ml/cavity
mit IP: (50 mmol/l Phosphatpuffer pH 7,4, 0,9 % NaCl, 1 % RSA [Rinderserumalbumin]) 2mal mit PBS waschen.

d) Testdurchführung: 0,1 ml/cavity
Probe (0 - 100 ng/ml HMLC bzw. SMLC) in IP zugeben und 2 h bei Raumtemperatur inkubieren. 2mal mit PBS waschen.
0,1 ml/cavity
Konjugat aus <HMLC>-PAK (Beispiel 5) und POD (Herstellung analog Beispiel 6 b) (Aktivität 0,1 U/ml) in IP zugeben und 2 h bei Raumtemperatur inkubieren. 2mal mit PBS waschen.
0,1 ml/cavity
Substratlösung (ABTS® 1,9 mmol/l in 0,1 mol/l Citratpuffer pH 4,4, 3,2 mmol/l Natriumperborat) 45 min bei Raumtemperatur inkubieren.
Wellenlänge
Messung bei 405/490 nm am ELISA-Reader

Die Ergebnisse sind aus Tabelle 2 zu ersehen.

Tabelle 2:

| Konzentration ng/ml | HMLC E | SMLC E |
|---|---|---|
| 0 | 0,000 | 0,000 |
| 10 | 0,091 | 0,000 |
| 25 | 0,288 | 0,000 |
| 50 | 0,339 | 0,000 |
| 100 | 0,913 | 0,000 |

**Beispiel 8**

Herstellung des SMLC-Immunadsorbers

Mit 15 %iger Salpetersäure und $H_2O$ gewaschenes Spherosil (Rhône Poulenc, XOC 005) wird nach Trocknen mit 10 % (v/v) 3(Triäthoxysilyl)propylamin in DMSO bei 85 °C über Nacht zu Spherosil-$NH_2$ umgesetzt. Nach der Umsetzung wird freies Reagenz mit DMSO und Isopropanol ausgewaschen und das Adsorbens bei 50 °C getrocknet.
Spherosil-$NH_2$ wird mit 10%iger Glutardialdehyd-Lösung pH 3,7 vermischt und bei 55 °C 2 h erwärmt. Die Suspension wird anschließend unter Vakuum über einen Glasfilter abgesaugt. Dann wird mit dem 7fachen Spherosil-Volumen an destilliertem Wasser gewaschen und mit dem 5fachen Volumen an 10 mmol/l Kaliumphosphat pH 8,0/0,1 mol/l Natriumchlorid nachgewaschen.
10 mg SMLC werden pro ml Spherosil in 10 mmol/l Kaliumphosphat pH 8,0/0,1 mol/l Natriumchlorid in ca. 50 % des eingesetzten Spherosil-Volumens in einem Rundkolben zugegeben. Der Kolben wird über Nacht bei Raumtemperatur am Rotationsverdampfer rotiert.
Nach Filtration wird das Spherosil mehrfach mit 0,9%iger NaCl-Lösung sowie Äthanolamin-Lösung nachgewaschen. Anschließend werden 3 Volumenteile Äthanolamin-Lösung zugegeben und 1 h bei Raumtemperatur inkubiert. Nach nochmaliger Filtration wird wieder mit NaCl-Lösung gewaschen.
Anschließend wird der Immunadsorber mit PBS auf pH 7,5 eingestellt und mit PBS/Natriumazid äquilibriert. Die Lagerung erfolgt bei 4 °C.

**Beispiel 9**

HMLC-Bestimmung über ELISA-Sandwich-Test mit monoklonalem Antikörper

Für die Bestimmung wurde ein Testsystem verwendet, welches aus einem immobilisierten <HMLC>-PAK (hergestellt nach Beispiel 5) und einem Konjugat aus einem <HMLC>-MAK (4D12 bzw. 1B10) und POD bestand (Konjugatherstellung analog Beispiel 6 b).

Festphase, Beschichtung, Nachbeladung und Durchführung der Bestimmung waren analog Beispiel 7.

Die Ergebnisse sind aus Fig. 1 zu ersehen.

Die Kurve zeigt, daß bei geringem Probenleerwert eine hohe Empfindlichkeit erreicht werden kann.

## Ansprüche

1. Spezifischer Antikörper gegen HMLC mit einer Kreuzreaktivität gegen SMLC von unter 5 %.

2. Spezifischer Antikörper nach Anspruch 1 mit einer Kreuzreaktivität gegen SMLC von unter 2 %.

3. Spezifischer Antikörper nach Anspruch 1 mit einer Kreuzreaktivität gegen SMLC von unter 1 %.

4. Spezifischer Antikörper nach Anspruch 1, 2 oder 3 dadurch gekennzeichnet, daß er erhältlich ist durch Immunisierung eines Säugetieres mit HMLC I und/oder HMLC II, Gewinnung des Rohserums und immunsorptiver Reinigung an einem SMLC-Immunadsorbens auf Silicatbasis.

5. Verfahren zur Herstellung von spezifischen Antikörpern gegen HMLC nach den Ansprüchen 1 bis 4 dadurch gekennzeichnet, daß ein Säugetier mit HMLC I und/oder HMLC.II immunisiert, das Rohserum gewonnen und einer immunsorptiven Reinigung an einem SMLC-Immunadsorbens auf Silicatbasis.

6. Verfahren nach Anspruch 5 dadurch gekennzeichnet, daß als Träger für das Immunadsorbens ein Silikagel verwendet wird.

7. Verfahren nach Anspruch 5 oder 6 dadurch gekennzeichnet, daß SMLC über einen bifunktionellen Linker an den Träger gekoppelt wird.

8. Verfahren nach Anspruch bis 7 dadurch gekennzeichnet, daß SMLC über Glutardialdehyd an den durch Aminogruppen aktivierten Träger gekoppelt wird.

9. Verfahren nach Anspruch bis 8 dadurch gekennzeichnet, daß SMLC über einen Abstand von nicht mehr als 20 Atomen an der Träger gebunden ist.

10. Reagenz zur Bestimmung von HMLC dadurch gekennzeichnet, daß es Antikörper gegen HMLC gemäß den Ansprüchen 1 bis 4 enthält.

11. Reagenz nach Anspruch 10 dadurch gekennzeichnet, daß es diese Antikörper in immobilisierter Form enthält.

12. Reagenz nach Anspruch 11 dadurch gekennzeichnet, daß es ein Konjugat aus HMLC-Antikörpern und einer bestimmbaren Gruppe enthält.

13. Reagenz nach Anspruch 10 oder 11 dadurch gekennzeichnet, daß es ein Konjugat aus einem monoklonalen Antikörper gegen HMLC und einer bestimmbaren Gruppe enthält.

14. Reagenz nach den Anspruch 13 dadurch gekennzeichnet, daß es ein Konjugat aus dem monoklonalen Antikörper 4D12 oder 1B10 und einer bestimmbaren Gruppe enthält.

15. Monoklonaler Antikörper 4D12, erhältlich aus der Hybridoma-Zellinie ECACC 88022503.

16. Monoklonaler Antikörper 1B10, erhältlich aus der Hybridoma-Zellinie ECACC 88022504.

17. Verfahren zur Bestimmung von HMLC dadurch gekennzeichnet, daß die Probe mit einem immobilisierten Antikörper gemäß den Ansprüchen 1 bis 4 und einem Konjugat aus einem monoklonalen Antikörper gegen HMLC und einer bestimmbaren Gruppe zusammengebracht wird, die Phasen getrennt werden und die bestimmbare Gruppe in einer der beiden Phasen bestimmt wird.

18. Verfahren nach Anspruch 17 dadurch gekennzeichnet, daß als monoklonaler Antikörper ein Antikörper, erhältlich aus der Zellinie ECACC 88022503 oder ECACC 88022504, verwendet wird.

Fig. 1